(19) Europäisches Patentamt
European Patent Office
Office européen des brevets

(11) Publication number: **0 055 045**
**B1**

(12) **EUROPEAN PATENT SPECIFICATION**

(45) Date of publication of patent specification: **12.09.84**

(51) Int. Cl.$^3$: **C 07 C 41/06,** C 07 C 43/04, B 01 J 29/04

(21) Application number: **81305734.6**

(22) Date of filing: **04.12.81**

(54) **Production of ethers.**

(30) Priority: **19.12.80 GB 8040779**

(43) Date of publication of application:
**30.06.82 Bulletin 82/26**

(45) Publication of the grant of the patent:
**12.09.84 Bulletin 84/37**

(84) Designated Contracting States:
**BE DE FR GB IT NL**

(56) References cited:
**DD-A- 133 661**
**GB-A-1 520 726**

(73) Proprietor: **IMPERIAL CHEMICAL INDUSTRIES PLC**
***Imperial Chemical House Millbank***
**London SW1P 3JF (GB)**

(72) Inventor: **Daniels, James Anthony**
**20A Kingsley Road**
**Frodsham Cheshire (GB)**
Inventor: **Stewart, Allan**
**20 Arran Drive**
**Frodsham Cheshire (GB)**

(74) Representative: **Martin, David Lincoln et al**
**Imperial Chemical Industries PLC Legal Department: Patents PO Box 6 Bessemer Road**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 055 045 B1

## Description

The present invention relates to the production of ethers, especially tertiary alkyl ethers.

It is known to produce ethers by reacting olefins and alcohols in the presence of suitable catalysts. For example, tertiary alkyl ethers may be prepared by reacting a tertiary olefin and an alcohol, e.g. isobutene and methanol to give methyl t-butyl ether, in the presence of catalysts such as mineral acids, e.g. sulphuric acid, and a range of solid catalysts such as heteropolytungstic or molybdic acids doped with phosphorus or boron, acidified alumina, and various acidified ion-exchange resins.

The use of mineral acid catalysts can give rise to corrosion problems which make it difficult to apply such catalysts on a commercial scale. Commercial processes are known using acidified ion-exchange catalysts, especially for the production of methyl t-butyl ether, which is particularly useful as a gasoline additive with high octane properties, but such catalysts often have poor thermal stability and this limits the reaction temperature which can be used, e.g. in many cases reaction temperatures not in excess of 80°C. The use of lower reaction temperatures can in turn result in long reaction times and low throughput per unit volume of reaction vessel.

Another problem which can arise with known processes is the formation of dialkyl ether byproducts deriving from the alcohol used.

Polish Patent No. 103379 describes a method of making methyl t-butyl ether by reacting methanol and isobutene in the presence of a catalyst comprising zeolite X or Y or a partially dealuminated, modified or ion-exchanged form thereof, the process being carried out in a vapour or liquid phase. Using zeolite Y in a vapour phase process at 120°C, a liquid product is obtained consisting of 8.1% methyl t-butyl ether and 91.8% methanol. The same zeolite is used in a liquid phase process at 160°C and a pressure of 31 atmospheres and a partially dealuminated zeolite Y is used at 125°C and a pressure of 23 atmospheres.

According to US Patent No. 2882244, zeolite X has a silica/alumina ratio in the range 2—3 and according to US Patent No. 3130007, zeolite Y has a silica/alumina ratio in the range 3—6.

East German Patent No. 133661 describes the reaction of alcohols and olefins to form ethers using a zeolite catalyst, the zeolite being either mordenite or faujasite. The activity and selectivity of these zeolites is generally rather low and appreciable amounts of side-products are also formed. In example 5, for instance, the amount of ether in the product is less than 40% while the amount of di-isobutene is nearly 60%.

We have now found that tertiary alkyl ethers may be prepared from tertiary olefins and alcohols in the presence of certain zeolite catalysts having higher activity and better selectivity than those used in the East Germany patent and under milder conditions than are described in the Polish patent. These catalysts and conditions allow the prouction of the tertiary alkyl ethers at high selectivity and in good yield, with low yields of undesirable by-product dialkyl ethers and oligomers of the olefin.

According to the present invention we provide a process for the production of an ether which comprises contacting an olefin and an alcohol with a catalyst comprising a zeolite having an $XO_2/Y_2O_3$ ratio equal to or greater than 10, wherein X is silicon and/or germanium, and Y is one or more of aluminium, iron, chromium, vandadium, molybdenum, arsenic, manganese, gallium or boron, the zeolite being predominantly in the hydrogen form.

The said zeolites have acid sites within zeolite pore systems active in the catalysis of tertiary alkyl ether formation. The entry port size in the preferred zeolites is such that reactants and products can move into and out of the pore systems, i.e. with a Lennard Jones diameter $\sigma$ (A) of from about 5.0 to 8.0 Å (the Lennard Jones diameter $\sigma$ (A) is defined by D W Breck in "Zeolite Molecular Sieves", Wiley Interscience, 1947, page 636).

The preferred zeolites for use as catalyst are based on $XO_2$ as silica ($SiO_2$) and $Y_2O_3$ as alumina ($Al_2O_3$). Suitable zeolites which may be employed as catalysts in the process of the invention include:

| | |
|---|---|
| Zeolite beta | (US 3308069) |
| ZSM—5 | (US 3702886; EPA 0011362) |
| ZSM—8 | (German OLS 2049755) |
| ZSM—11 | (US 3709979) |
| ZSM—12 | (US 3832449; EPA 0013630) |
| ZSM—23 | (US 4076842) |
| ZSM—35 | (US 4016245) |
| ZSM—43 | (EPA 0001695) |

| | |
|---|---|
| ZSM—48 | (EPA 0015132) |
| FU—1 | (UK 1563346) |
| FU—9 | (UK Appln. 8040781) |
| Nu—2 | (UK Appln. 8040782) |
| Nu—4 | (UK Appln. 8115407) |
| Nu—5 | (UK Appln. 8040395) |
| Nu—6(2) | (UK Appln. 8039685) |
| Nu—10 | (UK Appln. 8115410) |
| EU—1 | (European Appln. 81302343.9) |
| EU—2 | (GB 2077709A) |
| Zeolite omega | (UK 1178186) |
| Zeolite phi | (German OLS 2513682) |

The zeolites may be converted to their hydrogen form by methods which have been fully described in the prior art, for example by calcination of the as-made zeolite and subsequent acid exchange. If desired, the zeolites may be ion-exchanged or impregnated so as to comprise cations or oxides selected from the following, Cu, Ag, Ca, Sr, Zn, Cd, B, Al, Sn, Pb, V, P, Sb, Cr, Mo, W, Mn, Re, Fe, Co, Ni, noble metals and lanthanides. The formation of olefin oligomers may be further inhibited by treating the zeolite with a bulky-organic base, for example phenanthridine. It is believed that the base neutralises the surface acidic sites whilst being unable, because of its bulk, to enter the pore system of the zeolite.

Suitable olefins for use in the process of the invention include mono- or di-olefins containing from 4—16 carbon atoms, especially 4 to 9 carbon atoms, e.g. isobutene, 2-methylbut-1-ene, 2-methylbut-2-ene, 2-methylpent-1-ene, 2-methylpent-2-ene, 3-methylpent-2-ene, 2-methylhex-1-ene, 2-methylhept-1-ene and 2-methyloct-1-ene, or mixtures thereof.

Suitable alcohols for use in the process of the invention include primary and secondary alkanols containing from 1 to 12 carbon atoms, more preferably containing from 1 to 4 carbon atoms, e.g. methanol, ethanol, n-propanol, iso-propanol, and n-butanol. Also included are ether alcohols, e.g. $RO(CH_2CH_2O)_nH$ where R is H or hydrocarbyl and n is 1—20, e.g. 2-methoxyethanol.

The process of the invention is particularly applicable to the production of tertiary alkyl ethers containing a total of 5 to 10 carbon atoms from the corresponding tertiary olefins and alcohols, and especially to the production of methyl t-butyl ether from isobutene and methanol. The preferred zeolite catalysts for use in the production of MTBE are zeolites Nu—2, Nu—4, Nu—10 and beta.

The etherification reaction may be carried out in the vapour or liquid phase. The liquid phase reaction is typically carried out in a stirred and heated pressure vessel containing the reactants and catalyst. In the gas phase, the reactants may conveniently be passed through a heated tubular reactor containing the catalyst. This process readily lends itself to continuous production of the ether product.

The liquid phase and vapour phase processes may suitably be carried out at a temperature in the range from 50°C to 110°C, for example 70°C to 100°C. The reaction is normally conducted under atmospheric or superatmospheric pressure, e.g. at a pressure in the range 1 to 100 bars.

The molar ratio of the olefin to alcohol may vary widely but is suitably in the range from 0.5 to 5.0 moles of alkanol per mole of olefin.

For the liquid phase process, the proportion of catalyst in the reaction mixture may vary within very broad limits, but is suitably within the range from about 0.1 to about 5% by weight.

Under conditions suitable for the reaction of an alkanol and an olefin to yield tertiary alkyl ethers selectively, the zeolite catalyst is remarkably stable and does not itself suffer damage from degradation reactions which give materials having poor catalytic activity. By virtue of its nature, a zeolite can be rapidly recovered from the reaction mixture in liquid phase processes and can be reused in further batch operations.

Zeolites for use in the process of the invention, and methods for their preparation, have been described in the patent specifications referred to above. Inasmuch as some of these patent specifications are as yet unpublished, further details of zeolites Nu—2, Nu—4, Nu—5, Nu—6, Nu—10, EU—1, EU—2 and FU—9 are provided below.

Zeolite Nu—2 has a molar composition expressed by the formula:

$$0.5 \text{ to } 1.8\ R_2O : Y_2O_3 : \text{at least } 10\ XO_2 : 0 \text{ to } 100\ H_2O$$

wherein R is a monovalent cation or 1/n of a cation of valency, n, X is silicon and/or germanium, Y is one or more of aluminium, iron, chromium, vanadium, molybdenum, arsenic, manganese, gallium or boron, and $H_2O$ is water of hydration additional to water notionally present when R is H, and has an X-ray pattern substantially as set out in Tables 1 and 2 (as determined by standard technique using copper $K\alpha$ radiation). Table 1 shows X-ray data for zeolite Nu—2 as prepared, and Table 2 shows X-ray data for zeolite Nu—2 in the calcined Na—H form.

TABLE 1
Zeolite Nu-2 as made

| dA | 11.33 | 9.04 | 7.56 | 6.61 | 6.03 | 5.37 | 4.51 | 4.14 | 3.96 | 3.51 | 3.46 |
|---|---|---|---|---|---|---|---|---|---|---|---|
| 100I/Io | 23 vb | 3 | 4 vb | 3 | 3 vb | 5 vb | 2 | 23 | 100 vb* | 12 | 3 |

| dA | 3.38 | 3.31 | 3.10 | 3.02 | 2.93 | 2.91 | 2.68 | 2.59 |
|---|---|---|---|---|---|---|---|---|
| 100/I Io | 2 | 21 | 7 vb | 21 | 8 | 5 vb | 6 | 3 |

Vb = very broad diffraction peak
Vb* = very broad base but terminating in a very sharp peak

TABLE 2
Calcined sodium hydrogen Nu-2

| dA | 11.33 | 9.04 | 7.56 | 6.61 | 6.03 | 5.37 | 4.51 | 4.14 | 3.96 | 3.51 | 3.46 |
|---|---|---|---|---|---|---|---|---|---|---|---|
| 100I/Io | 22 vb | 17.5 | 4 vb | 3 | 3 vb | 0 | 2 | 12 | 100 vb* | 12 | 3 |

| dA | 3.38 | 3.31 | 3.10 | 3.02 | 2.93 | 2.91 | 2.68 | 2.59 |
|---|---|---|---|---|---|---|---|---|
| 100I/Io | 2 | 15 | 7 vb | 12 | 3 | 5 vb | 6 | 2 |

Within the above definition of chemical composition, the number of moles of $XO_2$ is typically in the range 10 to 100 and zeolite Nu—2 appears to be most readily formed in a state of high purity when the number of moles of $XO_2$ is in the range 25 to 50.

Zeolite Nu—2 may be prepared by reacting an aqueous mixture containing at least one oxide $XO_2$, at least one oxide $Y_2O_3$, and at least one alkylated or partially alkylated quaternary ammonium or phosponium or ternary sulphonium compound i.e. $(R_1R_2R_3R_4N)^+$ or $(R_1R_2R_3R_4P)^+$ or $(R_1R_2R_3S)^+$ hereinafter referred to as $Q^+$. $R_1$, $R_2$, $R_3$ and $R_4$ can be from two to four ethyl groups, the remainder can be H, $CH_3$ or $C_3H_7$. Alternatively precursors of the quaternary compound can be used e.g. triethylamine plus ethanol, or an ethyl halide or sulphate, in which case the precursor is preferably preheated in a solvent e.g. methyl ethyl ketone, prior to the addition of other reactants.

The reaction mixtures preferably have the following molar composition

$XO_2/Y_2O_3 \geqslant 10$, preferably 10 to 3000
$Ak^+/Q^+ = 0.15$ to 2.0
$H_2O/QZ = 30$—75
$OH^-/XO_2 = 0.1$ to 2.0
$H_2O/Ak^+ \geqslant 15$
$QZ/XO_2 = 0.02$ to 0.4

wherein X and Y are as above, $Ak^+$ is an alkali metal ion, or mixtures of such ions, which can include ammonium, and refers to free alkali, $OH^-$ includes free alkali and free quaternary ammonium hydroxide and Z is $OH^-$ or any acid radical. When Z is an acid radical an equivalent excess of free $Ak^-$ must be added as hydroxide in order to maintain the alkalinity of the reaction mixture. $Q^+$ is a quaternary ion of N, P or S.

The preferred quaternary compound is tetraethylammonium hydroxide.

Zeolite Nu—4 has a molar composition expressed by the formula:

$$5 \text{ to } 15\ M^1_2O : 0 \text{ to } 10\ Y_2O_3 : 100\ XO_2 : 0 \text{ to } 50\ H_2O$$

wherein $M^1$ is a monovalent cation or $^1/n$ of a cation of valency n, X is silicon or germanium, Y is aluminium, iron, chromium, vanadium, molybdenum, arsenic, antimony, manganese, gallium or boron, and $H_2O$ is water of hydration additional to water notionally present when $M^1$ is H, and has an X-ray pattern substantially as set out in Table 3 (as determined by standard technique using copper $K_\alpha$ radiation). Table 3 shows X-ray data for zeolite Nu—4 as prepared and in the calcined hydrogen form.

TABLE 3

| Zeolite Nu-4 (as made) dA | 100 $I/I_o$ | Zeolite Nu-4 (calcined H form) dA | 100 $I/I_o$ |
|---|---|---|---|
| 11.3 | 16 | — | — |
| 11.1 | 20 | 11.07 | 33 |
| 10.08 | 15 | 10.07 | 36 |
| 9.90 | 8 | 9.96 | 10 |
| 9.77 | 6 | 9.79 | 10 |
| — | — | 9.28 | 1 |
| 9.05 | 1 | 9.02 | 1.5 |
| — | — | 8.08 | 1 |
| 7.50 | 2 | 7.45 | 4 |
| 7.09 | 1 | 7.09 | 2 |
| 6.78 | 2 | 6.72 | 4 |
| 6.44 | 5 | 6.38 | 8 |
| 6.07 | 4 | 6.08 | 5 |
| 6.05 | 5 | 6.02 | 7 |
| 5.97 | 1 | 5.99 | 7 |
| 5.75 | 8 | 5.73 | 9 |
| 5.65 | 6 | 5.57 | 8 |
| 5.63 | 2 | — | — |
| 5.41 | 2 | 5.38 | 2 |
| 5.19 | 2 | 5.15 | 4 |
| 5.07 | 2 | 5.04 | 3 |
| 5.01 | 4 | 5.005 | 5 |

TABLE 3 (continued)

| Zeolite Nu-4 (as made) dA | 100 $I/I_o$ | Zeolite Nu-4 (calcined H form) dA | 100 $I/I_o$ |
|---|---|---|---|
| 4.915 | 1 | 4.899 | 1 |
| — | — | 4.725 | 1 |
| 4.629 | 9 | 4.610 | 9 |
| 4.558 | 1 | 4.560 | 1 |
| 4.495 | 2 | 4.490 | 1 |
| 4.475 | 2 | 4.470 | 2 |
| 4.386 | 13 | 4.380 | 14 |
| 4.291 | 10 | 4.280 | 13 |
| 4.124 | 13 | 4.103 | 3 |
| 4.104 | 4 | — | — |
| 4.039 | 6 | 4.022 | 7 |
| 3.950 | 1 | 3.950 | 2 |
| 3.880 | 100 | — | — |
| 3.850 | 69 | 3.869 | 100 |
| — | — | 3.836 | 73 |
| 3.743 | 51 | 3.764 | 35 |
| 3.730 | 50 | 3.735 | 54 |
| 3.678 | 27 | 3.662 | 29 |
| 3.649 | 22 | 3.604 | 4 |
| 3.629 | 5 | — | — |
| — | — | 3.500 | 5 |
| 3.466 | 12 | 3.469 | 10 |
| 3.364 | 6 | 3.368 | 7 |
| 3.332 | 9 | 3.342 | 10 |
| 3.329 | 4 | — | — |
| 3.273 | 4 | 3.265 | 3 |
| 3.267 | 4 | — | — |
| 3.260 | 4 | 3.260 | 12 |

Within the above definition of chemical composition, the number of moles of $Y_2O_3$ is typically in the range 0 to 10 and zeolite Nu—4 appears to be most readily formed in a state of high purity when the number of moles of $Y_2O_3$ is in the range 0 to 4.

Zeolite Nu—4 may be prepared by reacting an aqueous mixture comprising at least one oxide $XO_2$, at least one oxide $Y_2O_3$ and at least one polyalkylene polyamine having the formula:

$$\begin{matrix} R_1 & & R_4 \\ N & —[(CH_2)_x—N—R_3]_y—(CH_2)_x— & N \\ R_2 & & R_5 \end{matrix}$$

where x is in the range 2 to 6 and y is in the range from 0 to 10, an amine degradation product thereof, or a precursor thereof. In the polyamine, each of $R_1$ to $R_5$, independently, represents hydrogen or a $C_1$—$C_6$ alkyl group. When y is from 2 to 6, the $R_3$ substituents may be the same or different. When $y = 0$ then $x = 2$ to 5.

The reaction mixture can have the following molar ratios:

| | |
|---|---|
| $XO_2/Y_2O_3$ | $>\geqslant 10$ |
| $Q/XO_2$ | $= 0.01$ to 4.0 |
| $M^1OH/XO_2$ | $= 0$ to 2.0 |
| $H_2O/XO_2$ | $= 10$ to 200 |
| $M^2Z/XO_2$ | $= 0$ to 4.0 |

wherein X and Y are as above, $M^1$ is an alkali metal or ammonium or hydrogen, $M^2$ is an alkali metal or ammonium or hydrogen and can be the same as $M^1$ and Q is the aforesaid polyalkylene polyamine, amine degradation product thereof or a precursor thereof, or a related compound. $Z^-$ is a strong acid radical present as a salt of $M^2$ and may be added as a free acid to reduce the free $M^1OH$ level to a desired value. However, zeolite Nu—10 can be synthesised from a narrow range of molar ratios which falls within this much wider range for zeolite Nu—4 synthesis. Therefore if $SiO_2/Al_2O_3$ ratios between 70 and 300 are chosen, then to ensure that zeolite Nu—4 is obtained free of zeolite Nu—10 it is necessary to employ either low $H_2O/XO_2$ ratios or high $M^1OH/XO_2$ ratios or both.

The preferred ranges for preparing zeolite Nu—4 are as follows:

Range 1

| | | |
|---|---|---|
| | $XO_2/Y_2O_3$ | $= 20$ to 70 |
| | $Q/XO_2$ | $= 0.15$ to 4.0 |
| | $H_2O/XO_2$ | $= 15$ to 60 |
| | $M^1OH/XO_2$ | $= 0.01$ to 1.0 |
| | $M^2Z/XO_2$ | $= 0$ to 2.0 |

Range 2(a)

| | | |
|---|---|---|
| | $XO_2/Y_2O_3$ | $= 70$ to 120 |
| | $Q/XO_2$ | $= 0.10$ to 4.0 |
| | $M^2Z/XO_2$ | $= 0$ to 2.0 |
| if | $H_2O/XO_2$ | $= 20$ to 30 |
| then | $M^1OH/XO_2$ | $= 0.04$ to 1.0 |

2(b):

| | | |
|---|---|---|
| if | $H_2O/XO_2$ | $= 30$ to 40 |
| then | $M^1OH/XO_2$ | $= 0.1$ to 1.0 |

2(c):

| | | |
|---|---|---|
| if | $H_2O/XO_2$ | $= 40$ to 60 |
| then | $M^1OH/XO_2$ | $= 0.18$ to 1.0 |

Range 3

| | |
|---|---|
| $XO_2/Y_2O_3$ | = 120 to 300 |
| $Q/XO_2$ | = 0.05 to 4.0 |
| $M^2Z/XO_2$ | = 0 to 2.0 |
| $H_2O/XO_2$ | = 20 to 60 |
| $M^1OH/XO_2$ | = 0.08 to 1.0 |

Range 4

| | |
|---|---|
| $XO_2/Y_2O_3$ | = 300 to 800 |
| $Q/XO_2$ | = 0.02 to 4.0 |
| $M^2Z/XO_2$ | = 0 to 2.0 |
| $H_2O/XO_2$ | = 10 to 70 |
| $M^1OH/XO_2$ | = 0 to 1.0 |

Range 5

| | |
|---|---|
| $XO_2/Y_2O_3$ | = 800 to infinity (i.e. to no $Y_2O_3$) |
| $Q/XO_2$ | = 0.01 to 4.0 |
| $M^2Z/XO_2$ | = 0 to 2.0 |
| $H_2O/XO_2$ | = 10 to 80 |
| $M^1OH/XO_2$ | = 0 to 2.0 |

The preferred polyalkylene polyamines are triethylene tetramine and tetraethylene pentamine. Zeolite Nu—5 has a molar composition expressed by the formula:

$$0.5 \text{ to } 1.5\ R_2O : Y_2O_3 : \text{at least } 10\ XO_2 : 0 \text{ to } 2000\ H_2O$$

wherein R is a monovalent cation or $^1/n$ of a cation of valency n, X is silicon and or germanium, Y is one or more of aluminium, iron, chromium, vanadium, molybdenum, arsenic, manganese, gallium or boron, and $H_2O$ is water of hydration additional to water notionally present when R is H, and has an X-ray pattern substantially as set out in Table 4 (as determined by standard technique using copper $K_\alpha$ radiation). Table 4 shows X-ray data for zeolite Nu—5.

TABLE 4
X-ray diffraction data for Nu-5

| As-made Nu-5 | | Hydrogen Nu-5 | |
|---|---|---|---|
| dA | 100I/Io | dA | 100I/Io |
| 11.11 | 70 | 11.12 | 85 |
| 10.02 | 41 | 10.04 | 51 |
| 9.96 | 37 | 9.96 | 45 |
| 9.74 | 18 | 9.75 | 20 |
| 9.00 | 3 | 8.95 | 3 |
| 8.04 | 1 | 8.03 | 1 |
| 7.44 | 6 | 7.43 | 4 |
| 7.08 | 3 | 7.08 | 3 |
| 6.71 | 7 | 6.71 | 8 |
| 6.36 | 14 | 6.37 | 15 |
| 5.99 | 15 | 6.01 | 19 |
| 5.70 | 12 | | |
| 5.59 | 13 | 5.58 | 15 |
| 5.13 | 4 | 5.14 | 3 |
| 5.03 | 6 | 5.02 | 5 |
| 4.984 | 8 | 4.984 | 8 |
| 4.623 | 7 | 4.616 | 8 |
| 4.371 | 15 | 4.370 | 14 |
| 4.266 | 15 | 4.266 | 15 |

TABLE 4 (continued)
X-ray diffraction data for Nu-5

| As-made Nu-5 | | Hydrogen Nu-5 | |
|---|---|---|---|
| dA | 100I/Io | dA | 100I/Io |
| 4.095 | 14 | 4.095 | 9 |
| 4.014 | 11 | 4.022 | 12 |
| 3.859 | 100 | 3.859 | 100 |
| 3.821 | 70 | 3.825 | 68 |
| 3.749 | 39 | 3.755 | 32 |
| 3.725 | 54 | 3.731 | 48 |
| 3.643 | 31 | 3.652 | 28 |
| 3.598 | 4 | 3.601 | 4 |
| 3.484 | 7 | 3.484 | 6 |
| 3.358 | 10 | 3.355 | 9 |
| 3.315 | 12 | 3.315 | 11 |
| 3.054 | 12 | 3.054 | 12 |
| 2.994 | 13 | 2.991 | 15 |
| 2.979 | 13 | 2.979 | 12 |
| 2.015 | 8 | 2.015 | 10 |
| 1.996 | 8 | 1.994 | 10 |

Within the above definition of chemical composition, the number of moles of $XO_2$ is typically in the range 10 to 5000 and zeolite Nu—5 appears to be most readily formed in a state of high purity when the number of moles of $XO_2$ is in the range 45 to 100.

Zeolite Nu—5 may be prepared by reacting an aqueous mixture comprising at least one oxide $XO_2$, at least one oxide $Y_2O_3$ and at least one compound selected from pentaerythritol, dipentaerythritol and tripentaerythritol.

The reaction mixture preferably has the following molar composition:

| | | |
|---|---|---|
| $XO_2/Y_2O_3$ | 10 to 5000 | preferably 50 to 200 |
| $MOH/XO_2$ | 0.01 to 0.5 | preferably 0.10 to 0.25 |
| $Z^-/Y_2O_3$ | 0 to 5000 | preferably 10 to 100 |
| $A/Y_2O_3$ | 1 to 200 | preferably 1 to 50 |
| $H_2O/XO_2$ | 10 to 500 | preferably 15 to 300 |

wherein X and Y are as above, M is an alkali metal or ammonium, A is the aforesaid pentaerythritol compound and $Z^-$ is a strong acid radical present as a salt of M and may be added as a free acid to reduce the free $OH^-$ level to a desired value. M can be present as hydroxides or salts of inorganic or organic acids provided the $MOH/XO_2$ requirement is fulfilled.

The preferred pentaerythritol compound is pentaerythritol, itself.

Zeolite Nu—6(2) has a molar composition expressed by the formula:

$$0.5 \text{ to } 1.5\ R_2O : Y_2O_3 : \text{at least } 10\ XO_2 : 0 \text{ to } 2000\ H_2O$$

wherein R is a monovalent cation or $^1/n$ of a cation of valency n, X is silicon, and/or germanium, Y is one or more of aluminium, iron, chromium, vanadium, molybdenum, antimony, arsenic, manganese, gallium

or boron, and $H_2O$ is water of hydration, additional to water notionally present when R is H, and has an X-ray diffraction pattern substantially as set out in Table 5 (as determined by standard technique using copper Kα radiation).

TABLE 5 — ZEOLITE Nu-6(2)

| dA | 8.41 | 6.67 | 6.09 | 4.61 | 4.33 | ca 4.19 | ca 4.10 |
|---|---|---|---|---|---|---|---|
| 100I/Io | 45B | 42 | 15B | 27.5 | 100 | shoulder | |
| dA | 3.94 | 3.76 | 3.65 | 3.44 | 3.33 | 3.17 | 3.05 |
| 100I/Io | 2B | 11B | 15B | 27B | 76 | 15B | 9 |

Within the above definition of chemical composition, the number of moles of $XO_2$ is typically in the range 10 to 5000 and zeolite Nu—6(2) appears to be most readily formed in a state of high purity when the number of moles of $XO_2$ is in the range 20 to 1000.

Zeolite Nu—6(2) may be prepared by heating zeolite Nu—6(1) at a temperature in the range 200°C to 750°C, zeolite Nu—6(1) itself being made together with some zeolite Nu—6(2) by reacting an aqueous mixture containing at least one oxide $XO_2$, at least one oxide $Y_2O_3$ and a 4,4'-dipyridyl compound.

The reaction mixture preferably has the following molar composition:

| | | |
|---|---|---|
| $XO_2/Y_2O_3$ | 10 to 5000 | preferably 20 to 3000 |
| $MOH/XO_2$ | 0 to 1.0 | preferably 0.01 to 0.3 |
| $Z^-/Y_2O_3$ | 10 to 5000 | preferably 10 to 100 |
| $Q/Y_2O_3$ | 0.1 to 5000 | preferably 1 to 500 |
| $H_2O/XO_2$ | 10 to 500 | preferably 15 to 300 |
| $BOH/Y_2O_3$ | 0 to 500,000 | preferably 0 to 1000 |

wherein X and Y are as above, M is an alkali metal or ammonium, Q is the aforesaid 4,4'-bipyridyl compound and $Z^-$ is a strong acid radical present as a salt of M and may be added as a free acid to reduce the free $OH^-$ level to a desired value. M and/or Q can be present as hydroxides or salts of inorganic or organic acids provided the $MOH/XO_2$ requirement is fulfilled. BOH is an aliphatic or aromatic alcohol, preferably an alkanol. Whilst not essential, an alcohol improves crystallisation in viscous reaction mixtures.

The preferred bipyridyl compound is 4,4'-bipyridyl itself.

The preferred alcohol (BOH) is ethanol.

Zeolite Nu—10 has a molar composition expressed by the formula:

$$0.5 \text{ to } 1.5\ R_2O : Y_2O_3 : \text{at least } 60\ XO_2 : 0 \text{ to } 200\ H_2O$$

wherein R is a monovalent cation or $^1/n$ of a cation of valency n, X is silicon, and/or germanium, Y is one or more of aluminium, iron, chromium, vanadium, molybdenum, arsenic, antimony, manganese, gallium or boron, and $H_2O$ is water of hydration additional to water notionally present when R is H, and has an X-ray pattern substantially as set out in Table 6 (as determined by standard technique using copper Kα radiation).

TABLE 6

X-Ray Data of Zeolite Nu-10

| d(A) | I |
|---|---|
| 10.95±0.25 | m→s |
| 8.80±0.14 | w→m |
| 6.99±0.14 | w→m |
| 5.41±0.10 | w |
| 4.57±0.09 | w |
| 4.38±0.08 | vs |
| 3.69±0.07 | vs |
| 3.63±0.07 | vs |
| 3.48±0.06 | m→s |
| 3.36±0.06 | w |
| 3.31±0.05 | w |
| 2.78±0.05 | w |
| 2.53±0.04 | m |
| 2.44±0.04 | w |
| 2.37±0.03 | w |
| 1.88±0.02 | w |

vs = 60 to 100
s = 40 to 60
m = 20 to 40
w = 0 to 20

Within the above definition of chemical composition the number of moles of $XO_2$ is typically in the range 60 to 500. Zeolite Nu—10 appears to be most readily formed in a state of high purity when the number of moles of $XO_2$ is in the range 80 to 120.

Zeolite Nu—10 may be prepared by reacting an aqueous mixture containing at least one oxide $XO_2$, at least one oxide $Y_2O_3$ and at least one polyalkylene polyamine having the formula:

$$\begin{matrix} R_1 & & R_4 \\ N\text{—}[(CH_2)_x\text{—}N\text{—}R_3]_y\text{—}(CH_2)_x\text{—}N & & \\ R_2 & & R_5 \end{matrix}$$

where x is in the range 2 to 6 and y is in the range from 0 to 10, an amine degradation product thereof, or a precursor thereof. In the polyamine, each of $R_1$ to $R_5$, independently, represents hydrogen or a $C_1$—$C_6$ alkyl group. when y is from 2 to 6, the $R_3$ substituents may be the same or different. When $y = 0$ then $x = 2$ to 5.

The reaction mixture preferably has the following molar ratios:

$XO_2/Y_2O_3$ = 60 to 500, preferably 70 to 200, most preferred 80 to 150

$M^1OH/XO_2 = 10^{-8}$ to 1.0, preferably $10^{-6}$ to 0.25, most preferred $10^{-4}$ to 0.15

$H_2O/XO_2$ = 10 to 200, preferably 15 to 60, most preferred 30 to 50

$Q/XO_2$ = 0.5 to 4, preferably 0.1 to 1.0, most preferred 0.2 to 0.5

$M^2Z/XO_2$ = 0 to 4.0, preferably 0 to 1.0, most preferred 0 to 0.6

wherein X and Y are as above, $M^1$ is an alkali metal or ammonium or hydrogen, $M^2$ is an alkali metal or ammonium or hydrogen and can be the same as $M^1$ and Q is the aforesaid polyalkylene polyamine, amine degradation product thereof or a precursor thereof, or a related compound. $Z^-$ is a strong acid radical present as a salt of $M^2$ and may be added as a free acid to reduce the free $M^1OH$ level to a desired value.

The preferred polyalkylene polyamines are triethylene tetramine and tetraethylene pentamine.

Zeolite EU—1 has a molar composition expressed by the formula:

$$0.5 \text{ to } 1.5\ R_2O : Y_2O_3 : \text{at least } 10\ XO_2 : 0 \text{ to } 100\ H_2O$$

wherein R is a monovalent cation or $^1/n$ of a cation of valency n, X is silicon and/or germanium, Y is one or more of aluminium, iron, gallium or boron, and $H_2O$ is water of hydration additional to water notionally present when R is H, and has an X-ray pattern substantially as set out in Tables 7 and 8 (as determined by standard technique using copper $K\alpha$ radiation). Table 7 shows X-ray data for zeolite EU—1 as prepared, and Table 8 shows X-ray data for zeolite EU—1 in the calcined Na—H form.

TABLE 7

Zeolite EU-1 as freshly prepared

| d (A) | I/Io |
|---|---|
| 11.03 | Very Strong |
| 10.10 | Strong |
| 9.72 | Weak |
| 6.84 | Weak |
| 5.86 | Very Weak |
| 4.66 | Very Strong |
| 4.31 | Very Strong |
| 4.00 | Very Strong |
| 3.82 | Strong |
| 3.71 | Strong |
| 3.44 | Medium |
| 3.38 | Medium |
| 3.26 | Strong |
| 3.16 | Very Weak |
| 3.11 | Very Weak |
| 2.96 | Very Weak |
| 2.71 | Very Weak |
| 2.55 | Weak |
| 2.48 | Very Weak |
| 2.42 | Very Weak |
| 2.33 | Very Weak |
| 2.30 | Very Weak |
| 2.13 | Very Weak |

TABLE 8

Zeolite EU-1 in calcined Na—H form

| d (A) | I/Io |
|---|---|
| 11.11 | Very strong |
| 10.03 | Very strong |
| 9.78 | Weak |
| 7.62 | Weak |
| 6.84 | Medium |
| 6.21 | Very Weak |
| 5.73 | Weak |
| 4.87 | Very weak |
| 4.60 | Very strong |
| 4.30 | Very strong |
| 3.97 | Very strong |
| 3.77 | Strong |
| 3.71 | Weak |
| 3.63 | Very weak |
| 3.42 | Medium |
| 3.33 | Medium |
| 3.27 | Strong |
| 3.23 | Medium |
| 3.15 | Weak |
| 3.07 | Weak |
| 2.93 | Weak |
| 2.69 | Weak |
| 2.63 | Very weak |
| 2.57 | Very weak |
| 2.51 | Weak |
| 2.45 | Very weak |
| 2.41 | Very weak |
| 2.32 | Very weak |
| 2.29 | Very weak |
| 2.11 | Very weak |

Within the above definition of chemical composition, the number of moles of $XO_2$ is typically in the range 10 to 500 and zeolite EU—1 appears to be most readily formed in a state of high purity when the number of moles of $XO_2$ is in the range 20 to 300.

Zeolite EU—1 may be prepared by reacting an aqueous mixture comprising at least one oxide $XO_2$, at least one oxide $Y_2O_3$ and at least one alkylated derivative of a polymethylene $\alpha$-$\omega$ diamine having the formula:

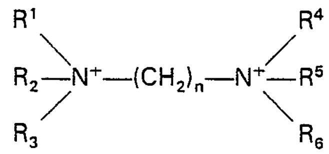

an amine degradation product thereof, or a precursor thereof, wherein n is in the range from 3 to 12 and $R_1$ to $R_6$ which may be the same or different, can be alkyl or hydroxyalkyl groups, containing from 1 to 8 carbon atoms and up to five of the groups $R_1$—$R_6$ can be hydrogen, the mixture having the molar composition:

| | | |
|---|---|---|
| $XO_2/Y_2O_3$ | at least 10, | preferably 10 to 150 |
| $(OH^-/XO_2$ | 0.1 to 6.0, | preferably 0.1 to 1.0 |
| $(M^++Q)/Y_2O_3$ | 0.5 to 100 | |
| $Q/(M^++Q)$ | 0.1 to 1.0 | |
| $H_2O/XO_2$ | 1 to 100 | |

wherein X and Y are as above, M is an alkali metal or ammonium, and Q is the aforesaid alkylated derivative of a polymethylene diamine, an amine degradation product thereof, or a precursor thereof, or a related compound.

M and/or Q can be present as hydroxides or salts of inorganic or organic acids provided the $OH^-/XO_2$ requirement is fulfilled.

Preferred alkylated polymethylene diamine starting materials include alylated hexamethylene diamines, especially methylated hexamethylene diamines, for example 1:6-N,N,N,N$^1$,N$^1$,N$^1$-hexamethyl hexamethylene diammonium salts (e.g. halide, hydroxide, sulphate, silicate, aluminate).

Zeolite EU—2 has a molar composition expressed by the formula:

$$0.5 \text{ to } 1.5\ R_2O : Y_2O_3 : \text{at least } 70\ XO_2 : 0 \text{ to } 100\ H_2O$$

wherein R is a monovalent cation or $^1/n$ of a cation of valency n, X is silicon and/or germanium, Y is one or more of aluminium, iron, gallium, or boron, and $H_2O$ is water of hydration additional to water notionally present when a is H, and has an X-ray pattern substantially as set out in Table 9 (as determined by standard technique using copper $K\alpha$ radiation).

TABLE 9

| Zeolite EU-2 | |
|---|---|
| Interplanar Spacings d (A) | Relative Intensity 100 $^I/Io$ |
| 11.74 | 17 |
| 10.13 | 14 |
| 6.33 | 7 |
| 5.85 | 7 |
| 4.33 | 5 |
| 4.18 | 86 |
| 3.89 | 100 |
| 3.69 | 7 |
| 3.37 | 7 |
| 3.08 | 5 |
| 2.85 | 18 |
| 2.09 | 5 |

Within the above definition of chemical composition, the number of moles of $XO_2$ is typically in the range 100 to 5000 and zeolite EU—2 appears to be most readily formed in a state of high purity when the number of moles of $XO_2$ is in the range 150 to 3000.

Zeolite EU—2 may be prepared by reacting an aqueous mixture comprising at least one oxide $XO_2$, at least one oxide $Y_2O_3$ and at least one alkylated derivative of a polymethylene $\alpha$-$\omega$ diamine having the formula:

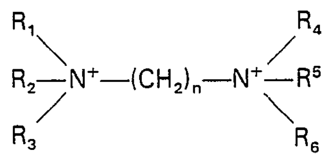

which by our definition is $Q^{2+}$ an amine degradation product thereof, or a precursor thereof, wherein n is in the range from 3 to 12, $R_1$ to $R_6$ which may be the same or different, can be alkyl or hydroxyalkyl groups containing from 1 to 8 carbon atoms and up to five of the groups $R_1$—$R_6$ can be hydrogen, the mixture having the molar composition:

| | | |
|---|---|---|
| $XO_2/Y_2O_3$ | at least 70, | preferably at least 150 |
| $OH^-/XO_2$ | 0.1 to 6.0 | preferably 0.1 to 1.0 |
| $(M^++Q)/Y_2O_3$ | 0.5 to 100 | |
| $Q/(M^++Q)$ | 0.1 to 1.0 | |
| $H_2O/XO_2$ | 1 to 100 | |

wherein X and Y are as above, M is an alkali metal or ammonium and Q is the aforesaid alkylated derivative of a polymethylene diamine, an amine degradation product thereof, or a precursor thereof, or a related compound.

M and/or Q can be present as hydroxides or salts of inorganic or organic acids provided that $OH^-/XO_2$ requirement is fulfilled.

Preferred alkylated polymethylene diamine starting materials include alkylated hexamethylene diamines, especially methylated hexamethylene diamines, for example 1:6-N,N,N,N$^1$,N$^1$,N$^1$-hexamethyl hexane-1,6-diammonium salts (e.g. halide, hydroxide, sulphate, silicate, aluminate).

Zeolite FU—9 has a molar composition expressed by the formula:

$$0.5 \text{ to } 1.5\ R_1O : Y_2O_3 : 15 \text{ to } 30\ XO_2 : 0 \text{ to } 500\ H_2O$$

wherein R is a monovalent cation or $^1/n$ of a cation of valency n, X is silicon and/or germanium, Y is one or more of aluminium, iron, chromium, vanadium, molybdenum, arsenic, manganese, gallium or boron, and $H_2O$ is water of hydration additional to water notionally present when R is H, and having an X-ray pattern substantially as set out in Table 10 (as determined by standard technique using copper $K\alpha$ radiation). Table 10 shows X-ray data for zeolite FU—9 as prepared.

Zeolite FU—9 may be prepared by reacting an aqueous mixture comprising at least one oxide $XO_2$, at least one oxide $Y_2O_3$ and at least one tetramethylammonium compound.

The reaction mixture preferably has the following molar composition:

| | | |
|---|---|---|
| $XO_2/Y_2O_3$ | 5 to 50 | preferably 10 to 30 |
| free $MO_2/XO_2$ | 0.1 to 1.0 | preferably 0.1 to 0.5 |
| $Z^-/Y_2O_3$ | 0 to 5000 | preferably 10 to 100 |
| $Q/Y_2O_3$ | 0.1 to 150 | preferably 1 to 50 |
| $H_2O/XO_2$ | 5 to 200 | preferably 10 to 30 |
| $Q = (TMA)_2 + xA$ | | |

wherein X and Y are as above, M is an alkali metal or ammonium, and Q is a mixture of TMA the tetramethylammonium compound, amine degradation product thereof or a precursor thereof, or a related compound, and A which is a trialkylamine and/or an alkanolamine or salt thereof, where x is equal to 0.2 to 2.0 moles and A preferably contains 1 to 12 carbon atoms. $Z^-$ is a strong acid radical present as a salt of M and may be added as a free acid to reduce the free $M_2O$ level to a desired value. M and/or Q can be present as hydroxides or salts of inorganic acids provided the $M_2O/XO_2$ requirement is fulfilled.

The preferred quaternary compound is a tetramethyl ammonium hydroxide.

For the efficient use of the reactants in methyl t-butyl ether production, it is important that any catalyst for the vapour phase reaction does not promote by-product formation. By virtue of its unique crystal structure, the channel system in our preferred zeolite,

TABLE 10

| | | | | | | | | | | |
|---|---|---|---|---|---|---|---|---|---|---|
| dA | 11.3 | 9.5 | 7.05 | 6.99 | 6.61 | 5.77 | 5.67 | 4.97 | 4.84 | 4.75 |
| 100I/Io | 7 | 100 | 21 | 22 | 19 | 13 | 3 | 8 | 1 | 2 |
| dA | 4.57 | 3.99 | 3.94 | 3.85 | 3.78 | 3.66 | 3.56 | 3.53 | 3.49 | 3.38 |
| 100I/Io | 2 | 52 | 37 | 18 | 32 | 14 | 40 | 55 | 52 | 10 |
| dA | 3.31 | 3.14 | 3.05 | 2.950 | 2.898 | 2.713 | 2.643 | 2.617 | 2.575 | 2.545 |
| 100I/Io | 15 | 21 | 8 | 9 | 5 | 3 | 4 | 1 | 3 | 1 |
| dA | 2.477 | 2.414 | 2.347 | 2.308 | 2.260 | 2.150 | 2.109 | 2.027 | 1.998 | |
| 100I/Io | 3 | 2 | 3 | 1 | 1 | 2 | 2 | 2 | 6 | |

Nu—10, limits the formation of oligomers of isobutene. Thus, a very high proportion of isobutene is converted to methyl t-butyl ether.

The invention is illustrated by the following Examples.

Examples 1—7

These examples illustrate the preparation of zeolite Nu—2 and its use as a catalyst in the production of methyl t-butyl ether (MTBE) from isobutene and methanol.

The synthesis mixture for the preparation of zeolite Nu—2 had the following molar composition:

$$1.35\ Na_2O,\ 3.14\ Q_2O,\ Al_2O_3,\ 29\ SiO_2,\ 311\ H_2O$$

8.7 g solid sodium hydroxide were dissolved in 250 g tetraethyl ammonium hydroxide (40% aqueous solution) followed by 16.4 g Kaiser SA alumina powder. Next 649 g colloidal silica were added with stirring. The resulting gel/slurry was crystallised to zeolite Nu—2 in a stirred autoclave after 6 days at 150°C. The washed dried product had the molar composition:

$$0.6\ Na_2O,\ 2.2\ Q_2O,\ Al_2O_3,\ 20\ SiO_2,\ 6\ H_2O$$

The product thus obtained was calcined at 450°C for 48 hours, followed by treating with normal hydrochloric acid for 24 hours, washing thoroughly with deionised water and calcined for 24 hours at 450°C. The product was zeolite H—Nu—2.

20 ml of methanol (0.5 mole) and 1 g of Nu—2 zeolite (prepared as above) were added to a glass flask in a stream of nitrogen. 20 ml (0.21 mole) of isobutene was condensed into the cooled flask. The mixture was put into an autoclave then heated with stirring at 90°C for one hour. After the reaction the autoclave was cooled to 0°C and the reaction mixture was analysed by gas chromatography.

The results are shown as Example 1 in Table 11 are as follows:

| | |
|---|---|
| Mole % conversion of isobutene | 92.3 |
| Mole % conversion of methanol | 39.4 |
| Selectivity to MTBE | 98.0 |

The procedure described for Example 1 was used in Examples 2—7. The results are also shown in Table 11.

TABLE 11

| Example | Catalyst | $SiO_2/Al_2O_3$ Ratio | Catalyst Conc % | Temp °C | Time hr | $CH_3OH$/Isobutene mole ratio | Mole % Conversion Isobutene | Mole % Conversion Methanol | Selectivity to M T B E |
|---|---|---|---|---|---|---|---|---|---|
| 1 | Nu-2 | 20 | 3.6 | 90 | 1 | 2.4 | 92.3 | 39.4 | 98.0 |
| 2 | Nu-2 | 20 | 0.9 | 90 | 1 | 2.4 | 89.5 | 38.2 | 99.0 |
| 3a | Nu-2 | 20 | 3.6 | 90 | 1 | 2.4 | 84.8 | 36.1 | 98.5 |
| 4b | Nu-2 | 20 | 3.6 | 90 | 1 | 2.4 | 82.0 | 35.0 | 98.4 |
| 5 | Nu-2 | 20 | 3.6 | 50 | 6 | 2.4 | 76.5 | 32.9 | 97.5 |
| 6 | Nu-2 | 20 | 3.6 | 50 | 18 | 2.4 | 87.8 | 38.6 | 98.3 |
| 7 | Nu-2 | 20 | 4.4 | 25 | 18 | 1.8 | 66.0 | 37.3 | 97.0 |
| 8 | ZSM-5 | 80 | 3.6 | 90 | 1 | 2.4 | 41.8 | 17.9 | 92.3 |
| 9 | ZSM-5 | 80 | 3.6 | 50 | 18 | 2.4 | 53.5 | 23.0 | 93.5 |
| 10 | FU1 | 28 | 3.6 | 90 | 1 | 2.4 | 16.5 | 7.2 | 89.0 |
| 11 | EU1 | 42 | 3.6 | 90 | 1 | 2.4 | 5.3 | 2.2 | 46.0 |
| 12 | ZSM-35 | 15 | 3.6 | 90 | 1 | 2.4 | 29.6 | 12.7 | 69.7 |
| 13 | EU2 | 150 | 3.6 | 90 | 1 | 2.4 | 30.0 | 12.8 | 96.5 |
| 14 | EU2 | 150 | 3.6 | 50 | 18 | 2.4 | 54.5 | 23.2 | 92.6 |
| 15 | Nu-4 | 40 | 3.6 | 90 | 1 | 2.4 | 28.4 | 11.9 | 88.5 |

TABLE 11 (continued)

| Example | Catalyst | $SiO_2/Al_2O_3$ Ratio | Catalyst Conc % | Temp °C | Time hr | $CH_3OH$/ Isobutene mole ratio | Mole % Conversion | | Selectivity to M T B E |
|---|---|---|---|---|---|---|---|---|---|
| | | | | | | | Isobutene | Methanol | |
| 16 | Zeolite Beta | 19 | 3.6 | 90 | 1 | 2.4 | 87.0 | 37.2 | 97.5 |
| 17 | FU9 | 11 | 3.6 | 90 | 1 | 2.4 | 8.5 | 3.6 | 87.8 |
| 18 | Nu-6(2) | 51 | 3.6 | 90 | 1 | 2.4 | 6.0 | 3.6 | 78.3 |

Notes

(a) Under 50 atmos of $CO_2$

(b) In the presence of 20 ml of hexane as solvent

(c) $\text{Catalyst conc} = \frac{\text{Wt of catalysts}}{\text{Wt of reactants}} \times 100$

Referring to Table 11, Examples 1—7 illustrate the use of zeolite Nu—2 as catalyst for the production of MTBE from isobutene and methanol.

Example 1 shows the high conversion and selectivity to MTBE in a short reaction time (1 hour) and at 90°C.

Example 2 demonstrates that a lower concentration of catalyst can be used without adversely affecting conversion and selectivity.

Example 3 shows that the reaction can be carried out under high pressure in the presence of an inert diluent (carbon dioxide), with only slight reduction in conversion and selectivity.

Example 4 shows that the reaction can be carried out in the presence of an inert solvent (hexane) with only slight reduction in conversion and selectivity.

Examples 5—7 show the reaction can be carried out at much lower temperature, although the reaction time is increased.

Examples 8—18

These examples illustrate the production of methyl t-butyl ether (MTBE) from isobutene and methanol using a range of zeolites as catalysts. The preparation of the zeolites is described in the relevent patent specifications (to which reference has already been made). The as-made zeolites were calcined and exchanged and calcined as described in Example 1.

The results, shown in Table 11, illustrate that zeolite beta (Example 16) and zeolite Nu—(2) (Examples 1—7) are the most effective catalysts.

Example 19

This example illustrates the production of ethyl t-butyl ether (ETBE) from isobutene and ethanol.

20 ml of ethanol (0.5 mole) and 1 g of Nu—2 zeolite were added to a glass flask in a stream of nitrogen. 20 ml (0.21 mole) of isobutene was condensed into the cooled flask. The mixture was put into an autoclave then heated with stirring at 90°C for one hour. After the reaction the autoclave was cooled to 0°C and the reaction mixture was analysed by gas chromatography.

The results were as follows:—

| | |
|---|---|
| Mole % conversion of isobutene | 65.5 |
| Mole % conversion of ethanol | 43.3 |
| Selectivity ot ETBE | 97.0 |

Example 20

This example illustrates the production of methyl 3-methylpentyl ether (M3MPE) from 3-methylpent-2-ene and methanol using zeolite Nu—2 as catalyst.

10 ml of methanol (0.25 mole), 10 ml of 3-methylpent-2-ene (0.082 mole) and 1 g Nu—2 zeolite were added to a glass flask in a stream of nitrogen. The mixture was put into an autoclave then heated to 90°C for one hour. After the reaction the autoclave was cooled to 20°C and the reaction mixture was analysed by gas chromatography.

The results were as follows:—

| | |
|---|---|
| Mole % conversion of 3-methyl-2-pentene | 21.2 |
| Mole % conversion of methanol | 7.7 |
| Selectivity to M3MPE | 94.5 |

Example 21

This example illustrates the production of ethylene glycol methyl t-butyl ether (EGMTBE) from isobutene and 2-methoxyethanol using zeolite Nu—2 as catalyst.

20 ml of 2-methoxyethanol (0.25 mole), 20 ml of isobutene (0.21 mole) and 0.25 g Nu—2 zeolite were added to a glass flask in a stream of nitrogen. The mixture was put into an autoclave then heated to 90°C for one hour. After the reaction, the mixture was analysed by gas chromatography.

The results were as follows:—

| | |
|---|---|
| Mole % conversion of isobutene | 42.3 |
| Mole % conversion of 2-methoxyethanol | 36.6 |
| Selectivity to EGMTBE | 96.5 |

Examples 22—24

1 gm of Nu—2 zeolite was powdered and packed into a glass tube. The tube and contents were heated by a furnace to 90°C whilst passing a continuous stream of methanol vapour and isobutene over the zeolite bed (mole ratio 2:1). The products from the reaction were collected at hourly intervals and analysed by gas chromatography.

The results, shown as Example 22 in Table 12, are as follows:—

| | |
|---|---|
| Selectivity to MTBE | 65—80% |
| Selectivity to Di-isobutene | 20—30% |
| Catalytic activity (g MTBE/g catalyst/hr) | 0.06—0.12 |

The procedure described for Example 22 was used in Examples 23 and 24 except that the temperature was lowered to 70°C then 50°C as shown in Table 12. Examples 22—24 demonstrate that selectivity to MTBE increases as the temperature is lowered.

Example 25

1 g of zeolite Nu—2 prepared as described in Example 1 was soaked in the bulky amine phenanthridine (20 ml) for 24 hours then filtered, washed well with hexane and dried on a vacuum line at 100°C. The amine treated zeolite was then packed into a glass tube as described in Example 22 and evaluated for the synthesis of MTBE in a flow reactor at 90°C.

The results are shown in Table 12 and demonstrate that at 90°C the bulky amine ion-exchanged onto the surface of the zeolite inhibits the formation of di-isobutene and improves the overall selectivity to MTBE without loss in catalyst activity.

Examples 26—30

These examples illustrate the production of methyl tertiary butyl ether from isobutene and methanol using a range of zeolites in the flow reactor. The preparation of the zeolites is described in the relevant patent specification (to which reference has already been made). The as-made zeolites were calcined and exchanged and calcined as described in Example 1.

TABLE 12

| Example | Catalyst | $SiO_2/Al_2O_3$ ratio | Temp °C | MeOH Isobutene mole ratio | Selectivity to MTBE % | Selectivity to Di-isobutene % | Activity g MTBE/ g cat/hr. |
|---|---|---|---|---|---|---|---|
| 22 | Nu-2 | 20 | 90 | 2 | 65—80 | 20—30 | 0.06—0.12 |
| 23 | Nu-2 | 20 | 70 | 2 | 85—95 | 0—5 | 0.08—0.13 |
| 24 | Nu-2 | 20 | 50 | 2 | 90—95 | 0—5 | 0.04—0.08 |
| 25 | Nu-2/phen-anthridine | 20 | 90 | 2 | 80—90 | 10—20 | 0.06—0.12 |
| 26 | Beta | 19 | 90 | 2 | 650—75 | 20—35 | 0.04—0.08 |
| 27 | Nu-10 | 90 | 90 | 2 | 85—95 | 5—15 | 0.06—0.09 |
| 28 | Nu-4 | 40 | 90 | 2 | 80—90 | 5—15 | 0.04—0.07 |
| 29 | Nu-4 | 40 | 70 | 2 | 95—100 | 0—5 | 0.10—0.12 |
| 30 | ZSM-5 | 80 | 90 | 2 | 85—95 | 0—10 | 0.02—0.06 |

The results, as shown in Table 12, illustrate that zeolites Nu—4 and Nu—10 are particularly effective catalysts.

## Claims

1. A process for the production of an ether which comprises contacting an olefin and an alcohol at a temperature in the range of 50°C to 110°C with a catalyst comprising a zeolite having an $XO_2/Y_2O_3$ ratio equal to or greater than 10, wherein X is silicon and/or germanium and Y is one or more of aluminium, iron, chromium, vanadium, molybdenum, arsenic, manganese, gallium or borom, the zeolite being predominantly in the hydrogen form.

2. A process according to claim 1 wherein the zeolite has been treated with a bulky organic base.

3. A process according to claim 1 or claim 2 wherein the olefin is a mono- or di-olefin containing from 4 to 16 carbon atoms.

4. A process according to claim 3 wherein the olefin contains 4 to 9 carbon atoms.

5. A process according to any one of the preceding claims wherein the alcohol is a primary or secondary alkanol containing from 1 to 12 carbon atoms.

6. A process according to claim 5 wherein the alcohol contains from 1 to 4 carbon atoms.

7. A process according to any one of claims 1 to 4 wherein the alcohol is an ether alcohol of the formula:

$$RO(CH_2CH_2O)_nH$$

where R is H or hydrocarbyl and n is 1—20.

8. A process according to any one of claims 1 to 6 wherein isobutene and methanol are reacted to form methyl t-butyl ether.

9. A process according to any one of the preceding claims wherein the zeolite is selected from zeolites Nu—2, Nu—4, Nu—10 and beta.

## Patentansprüche

1. Verfahren zur Herstellung eines Ethers, bei dem ein Olefin und ein Alkohol bei einer in dem Bereich von 50°C bis 110°C liegenden Temperatur mit einem Katalysator in Kontakt gebracht werden, der aus einem Zeolith mit einem $XO_2/Y_2O_3$-Verhältnis, das gleich oder größer als 10 ist, besteht, worin X Silicium und/oder Germanium ist und Y ein oder mehr als ein aus Aluminium, Eisen, Chrom, Vanadium, Molybdän, Arsen, Mangan, Gallium und Bor ausgewähltes Element ist, wobei der Zeolith überwiegend in der Wasserstofform vorliegt.

2. Verfahren nach Anspruch 1, bei dem der Zeolith mit einer sperrigen organischen Base behandelt worden ist.

3. Verfahren nach Anspruch 1 oder 2, bei dem das Olefin ein 4 bis 16 Kohlenstoffatome enthaltendes Mono- oder Diolefin ist.

4. Verfahren nach Anspruch 3, bei dem das Olefin 4 bis 9 Kohlenstoffatome enthält.

5. Verfahren nach einem der vorhergehenden Ansprüche, bei dem der Alkohol ein primäres oder sekundäres Alkanol ist, das 1 bis 12 Kohlenstoffatome enthält.

6. Verfahren nach Anspruch 5, bei dem der Alkohol 1 bis 4 Kohlenstoffatome enthält.

7. Verfahren nach einem der Ansprüche 1 bis 4, bei dem der Alkohol ein Etheralkohol der Formel:

$$RO(CH_2CH_2O)_nH$$

ist, worin R H oder eine Kohlenwasserstoffgruppe ist und n 1 bis 20 ist.

8. Verfahren nach einem der Ansprüche 1 bis 6, bei dem Isobuten und Methanol zur Reaktion gebracht werden, um Methyl-t-butylether zu bilden.

9. Verfahren nach einem der vorhergehenden Ansprüche, bei dem der Zeolith aus den Zeolithen Nu—2, Nu—4, Nu—10 und Beta ausgewählt ist.

## Revendications

1. Procédé de production d'un éther qui comprend la mise en contact d'un oléfine et d'un alcool à une température de l'intervalle de 50°C à 110°C avec un catalyseur comprenant une zéolite présentant un rapport $XO_2/Y_2O_3$ égal ou supérieur à 10, où X représente le silicium et/ou le germanium et Y représente l'un ou plusieurs d'entre l'aluminium, le fer, le chrome, le vanadium, le molybdène, l'arsenic, le manganèse, le gallium et le bore, la zéolite se trouvant principalement sous la forme hydrogène.

2. Procédé suivant la revendication 1, dans lequel la zéolite a été traitée au moyen d'une base organique volumineuse.

3. Procédé suivant la revendication 1 ou 2, dans lequel l'oléfine est une mono- ou dioléfine comptant 4 à 16 atomes de carbone.

4. Procédé suivant la revendication 3, dans lequel l'oléfine compte 4 à 9 atomes de carbone.

5. Procédé suivant l'une quelconque des revendications précédentes, dans lequel l'alcool est un alcanol primaire ou secondaire comptant 1 à 12 atomes de carbone.

6. Procédé suivant la revendication 5, dans lequel l'alcool compte 1 à 4 atomes de carbone.

7. Procédé suivant l'une quelconque des revendications 1 à 4, dans lequel l'alcool est un éther-alcool de formule:

$$RO(CH_2CH_2O)_nH$$

où R représente H ou un radical hydrocarbyle et n a une valeur de 1 à 20.

8. Procédé suivant l'une quelconque des revendications 1 à 6, dans lequel l'isobutène et le méthanol sont mis à réagir pour former l'éther méthylique du t-butanol.

9. Procédé suivant l'une quelconque des revendications précédentes, dans lequel la zéolite est choisie entre les zéolites Nu—2, Nu—4, Nu—10 et bêta.